(19) Europäisches Patentamt
European Patent Office
Office européen des brevets

(11) EP 0 904 766 A2

(12) # EUROPÄISCHE PATENTANMELDUNG

(43) Veröffentlichungstag:
**31.03.1999 Patentblatt 1999/13**

(51) Int. Cl.$^6$: **A61K 6/083**

(21) Anmeldenummer: **98118365.0**

(22) Anmeldetag: **29.09.1998**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **29.09.1997 DE 19742981**

(71) Anmelder: **ESPE Dental AG**
**82229 Seefeld (DE)**

(72) Erfinder: **Bissinger, Peter**
**86415 Mering (DE)**

(74) Vertreter:
**Freiherr von Wittgenstein, Arved, Dr. et al**
**Patentanwälte Abitz & Partner**
**Postfach 86 01 09**
**81628 München (DE)**

(54) **Durch ROMP härtbare Dentalmassen**

(57) Die Erfindung betrifft Dentalmassen, enthaltend

(a) 5 bis 70 Gew.-%, bezogen auf (a) + (b) + (d), polymerisierbare Monomere und/oder Polymere,
(b) 0 bis 95 Gew.-%, bezogen auf (a) + (b) + (d), Füllstoffe,
(c) 0,01 bis 15 Gew.-%, bezogen auf (a), mindestens eines Initiators oder eines Initiatorsystems,
(d) 0 bis 95 Gew.-%, bezogen auf (a) + (b) + (d), übliche Hilfsstoffe, einschließlich Pigmente, röntgenopake Zusatzstoffe und/oder Thixotropie-Hilfsmittel,

dadurch gekennzeichnet, daß der Bestandteil (a) eine solche chemische Struktur aufweist, daß die Teil- oder Endhärtung der Dentalmasse durch Ring-Öffnungs-Metathese-Polymerisation erfol-gen kann.

Die Dentalmassen zeichnen sich durch einen raschen Polymerisationsverlauf aus, der zu teil- oder endgehärteten Materialien führt, welche einen geringen Volumenschrumpf, eine geringe Abrasionsneigung und gute mechanische Eigenschaften aufweisen.

EP 0 904 766 A2

## Beschreibung

Die vorliegende Erfindung beschreibt polymerisierbare Massen für dentale Zwecke, die durch Ring-Öffnungs-Metathese-Polymerisation (ROMP) gehärtet oder teilgehärtet werden können.

In polymerisierbaren Dentalmassen werden bislang vonwiegend ethylenisch ungesättigte Monomere, bevorzugt Methacrylat- und Acrylatmonomere verwendet. Besonders häufig wird dabei das von Bowen beschriebene 2,2-Bis[4,1-phenylenoxy(2-hydroxy-3,1-propandiyl)-methacrylsäureester]-propyliden (Bis-GMA) [US-A-3 066 112] eingesetzt. Mischungen dieses Methacrylats mit Triethylenglykoldimethacrylat (TEGDMA) dienen auch heute noch als Standardrezeptur für dentale plastische Direkt-Füllungswerkstoffe. Die Aushärtung derartiger Massen beruht auf einer radikalischen Polymerisationsreaktion, welche durch entsprechend aktivierte radikalbildende Initiatoren gestartet wird. Problematisch hierbei ist der bei der Polyerisation auftretende nachteilige Polymerisationsschrumpf. Dieser kann beispielsweise bei der Anwendung als Füllungsmaterial zur Bildung von Verfärbungen am Kavitätenrand des Zahnes oder sogar zur Entstehung von Randspalten mit anschließendem Sekundärkariesrisiko führen.

Darüber hinaus gibt es in der Literatur noch Hinweise auf kationisch aushärtbare Dentalmassen [R. Bowen, J Dent Res (1956) 35, 360-379], [AT-A-204 687], die aber aufgrund von zu langen Aushärtezeiten und schlechten mechanischen Eigenschaften der ausgehärteten Materialien nicht erfolgreich eingesetzt werden konnten.

Aufgabe der vorliegenden Erfindung ist es, polymerisierbare Massen für zahnärztliche und zahntechnische Anwendungen bereitzustellen, die auf einem Polymerisationsprinzip beruhen, welches zu rasch teil- oder endhärtbaren Materialien führt, die einen geringen Polymerisationsschrumpf bei gleichzeitig sehr guten mechanischen Eigenschaften aufweisen.

Gelöst wird die Aufgabe durch Bereitstellung von Dentalmassen, die

(a) 5 bis 70 Gew.-%, vorzugsweise 15 bis 60 Gew.-%, bezogen auf (a) + (b) + (d) , polymerisierbare Monomere und/oder Polymere,
(b) 0 bis 95 Gew.-%, vorzugsweise 40 bis 80 Gew.-%, bezogen auf (a) + (b) + (d) , Füllstoffe,
(c) 0,01 bis 15 Gew.-%, vorzugsweise 1,0 bis 15 Gew.-%, insbesondere 2,0 bis 10 Gew.-%, bezogen auf (a), mindestens eines Initiators oder eines Initiatorsystems,
(d) 0 bis 95 Gew.-%, vorzugsweise 0 bis 30 Gew.-%, insbesondere 5 bis 30 Gew.-%, bezogen auf (a) + (b) + (d) , übliche Hilfssstoffe, einschließlich Pigmente, röntgenopake Zusatzstoffe und/oder Thixotropie-Hilfsmittel

enthalten, dadurch gekennzeichnet, daß der Bestandteil (a) eine solche chemische Struktur aufweist, daß die Teil- oder Endhärtung der Dentalmassen durch Ring-Öffnungs-Metathese-Polymerisation (ROMP) erfolgen kann.

Die Ring-Öffnungs-Metathese-Polymerisation ist literaturbekannt und wird seit einigen Jahren auch industriell eingesetzt [Comprehensive Polymer Sci.; 4, 109-142]. Problematisch für ein breites Anwendungsfeld dieser Polymerisationsmethode waren bislang die Sauerstoff- und Feuchtigkeitsempfindlichkeit der dazu nötigen Katalysatoren [J. Feldman, R.R. Schrock; Prog. Inorg.Chem. (1991), 39, 1]. Mittlerweile gibt es aber eine Reihe von Katalysatoren für die ROMP, welche diese Nachteile kaum oder gar nicht mehr aufweisen [WO-9623829 A1]. Auch photochemisch aktivierbare Katalysatoren für eine lichthärtende ROMP sind inzwischen zugänglich [P.A. van der Schaaf, A. Hafner, A. Mühlebach; Angew. Chem. (1996), 108, 1974-1977].

Es wurde nun überraschenderweise gefunden, daß sich die ROMP zusammen mit diesen neueren Katalysatoren in hervorragender Weise für die Herstellung einer Vielzahl von Massen eignet, die für dentale Zwecke eingesetzt werden können. Damit sind z.B. polymerisierbare Füllungsmaterialien, Befestigungszemente, Bondingmischungen, Inlays, Onlays, Verblendschalen, provisorische Kronen- und Brückenmaterialien, zahntechnische Werkstoffe, Modellmatenialien sowie Abformmaterialien gemeint.

Als Monomere bzw. Polymere gemäß Bestandteil (a) können Verbindungen der folgenden allgemeinen Formel eingesetzt werden:

$$M\text{-}A_n$$

wobei

M gleich H oder ein linearer, verzweigter, cyclischer oder polycyclischer organischer oder metallorganischer Rest ist. Organische Reste können $C_1$-$C_{30}$-Alkyl, $C_6$-$C_{20}$-Aryl, $C_7$-$C_{30}$-Alkaryl oder $C_3$-$C_{30}$-Cycloalkyl mit 0-10 Heteroatomen aus der Gruppe N, O, Si, P, S und einer Anzahl von n Anknüpfungspunkten für A sein. Metallorganische Reste enthalten neben den oben genannten organischen Resten zusätzlich lineare, verzweigte, cyclische oder polycycli-sche Gerüste anorganischer Natur.

Bevorzugte Reste M können

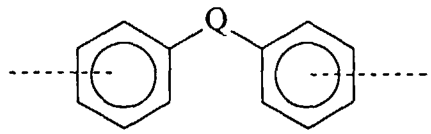

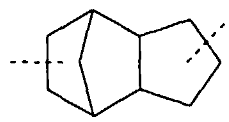

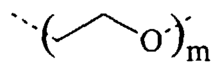

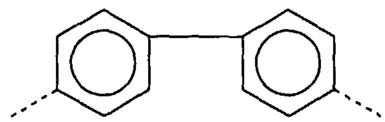

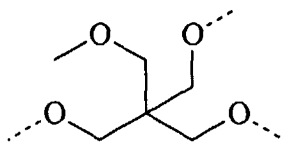

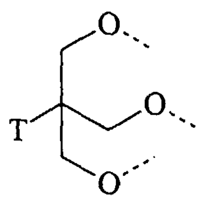

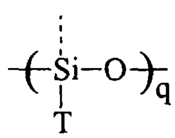

------- = Anknüpfungsstellen für A

sein, mit der Maßgabe, daß Q gleich O, S, $SO_2$ oder ein linearer, verzweigter oder cyclischer $C_1$-$C_{20}$-Alkylenrest ist, der auch fluoriert sein kann, m eine ganze Zahl von 1-20 ist, T ein linearer, verzweigter oder cyclischer gesättigter oder ungesättigter $C_1$-$C_{20}$-Kohlenwasserstoffrest ist und q eine ganze Zahl von 3-20 ist.

A ist ein ungesättigter cyclischer oder polycyclischer organischer Rest der allgemeinen Formel

C-D,

wobei C gleich H oder ein linearer, verzweigter oder cyclischer gesättigter oder ungesättigter organischer $C_1$-$C_{20}$-Rest mit 0-10 Heteroatomen aus der Gruppe N, O, Si, P, S und 0-10 Carbonylgruppen ist und

D ein Cyclobutenyl-, Cyclopentenyl- oder ein an bezeichneter und wahlweise zusätzlich an einer weiteren Stelle im Ringsystem ungesättigter Rest der allgemeinen Formel

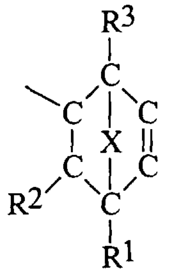

ist, in welcher bedeuten:

R[1], R[2], R[3] H oder einen linearen, verzweigten oder cyclischen gesättigten oder ungesättigten organischen $C_1$-$C_{20}$-Reste mit 0-10 Heteroatomen der Gruppe N, O, Si, P, S und 0-10 Carbonylgruppen, und
X O, NH, S bzw. einen gesättigten oder ungesättigten $C_1$-$C_{30}$-Kohlenwasserstoffrest.

Besonders bevorzugte Verbindungen, welche durch ROMP polymerisiert werden können, sind:

I) 2,2-Bis-{4,1-phenylenoxy-3,1-propandiyl-bicyclo[2.2.1]hept-2-enyl-6-carboxy}-propyliden

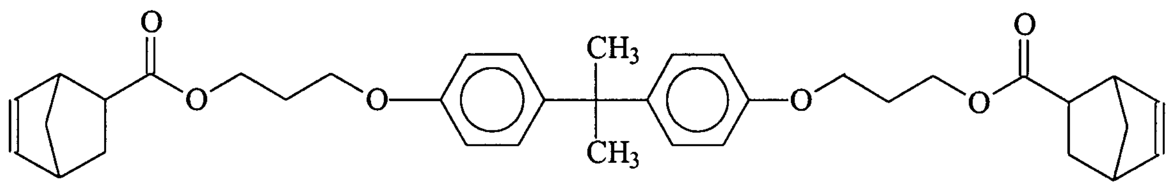

II) 2,2-Bis-{4,1-phenylenoxy-3,1-propandiyl-7-oxa-bicyclo[2.2.1]hept-2-enyl-6-carboxy}-propyliden

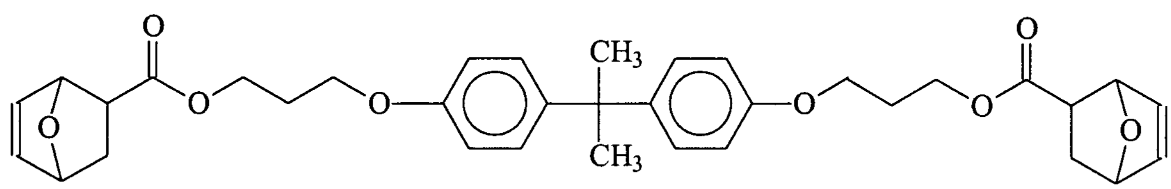

III) Bis-{methandiyl-oxy-3,1-propandiyl-bicyclo[2.2.1]hept-2-enyl-6-carboxyl}tricyclo[5.2.1.$0^{2,6}$]-decan

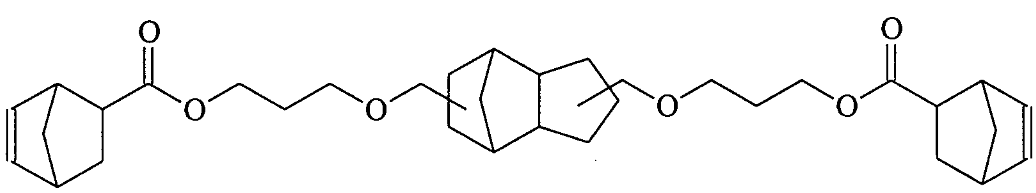

IV) Bis-{methandiyl-oxy-3,1-propandiyl-7-oxa-bicyclo-[2.2.1]hept-2-enyl-6-carboxyl}tricyclo[5.2.1.$0^{2,6}$]-decan

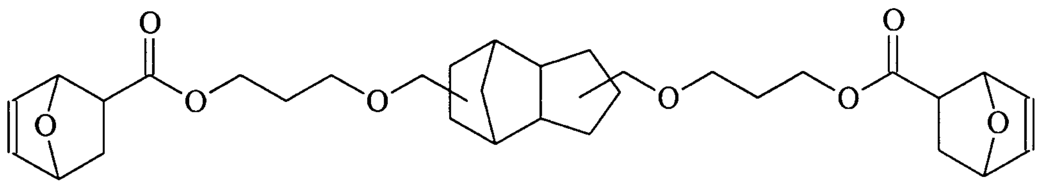

V) 1,1,1-Tris{methandiyl-bicyclo[2.2.1]hept-2-enyl-6-carboxy}propan

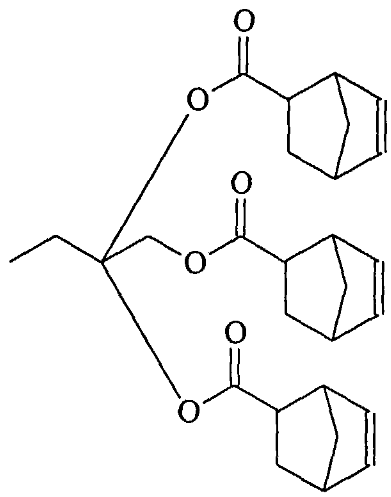

VI) 1,1,1-Tris{methandiyl-7-oxa-bicyclo[2.2.1]hept-2-enyl-6-carboxy}propan

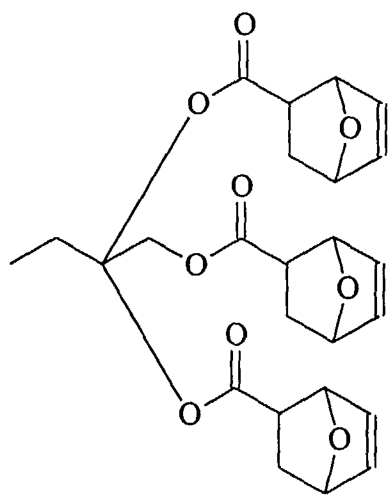

VII) 1,1,1-Tris{methandiyl-oxy-bis(ethandiyloxy)-bicyclo[2.2.1]hept-2-enyl-6-carboxy}propan

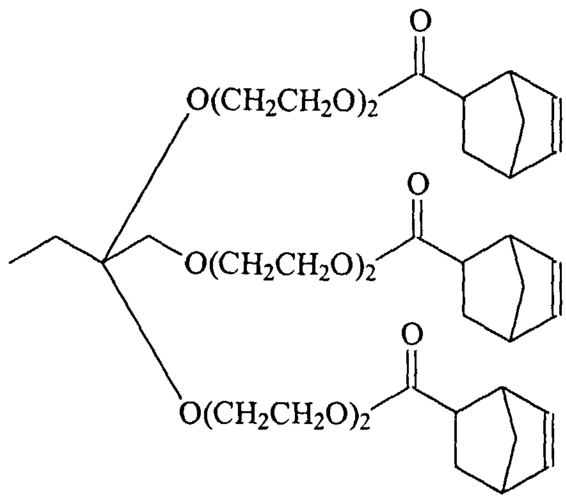

VIII) 1,1,1-Tris{methandiyl-oxy-bis(ethandiyloxy)-7-oxa-bicyclo[2.2.1]hept-2-enyl-6-carboxy}propan

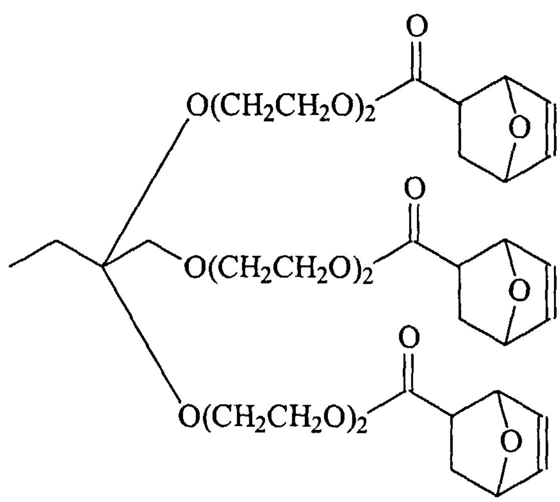

IX) α,ω-Bis{bicylo[2.2.1]hept-2-enyl-6-carboxy}-polytetrahydrofuran

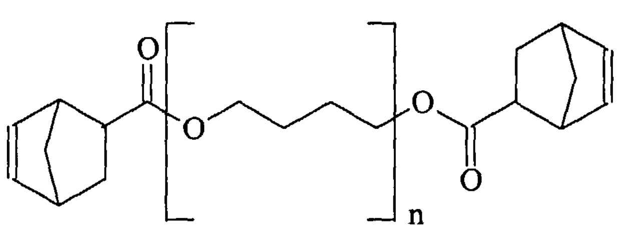

X) α,ω-Bis{7-oxa-bicylo[2.2.1]hept-2-enyl-6-carboxy}-polytetrahydrofuran

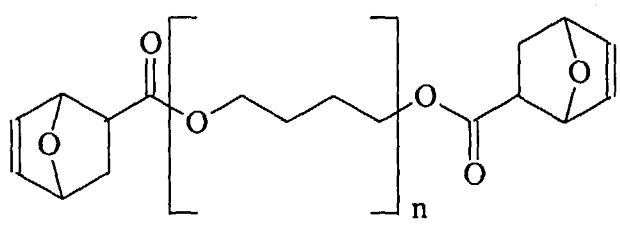

XI) 7-Oxabicyclo[2.2.1]hept-2-ene-5,6dicarbonsäureanhydrid

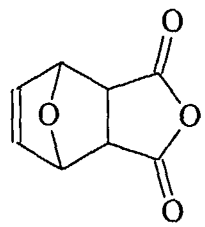

Die hier beschriebenen Verbindungen können in den offenbarten Dentalmassen entweder alleine oder in Kombination mit Co-Monomeren eingesetzt werden. Diese Co-Monomere können Verbindungen sein, die mindestens einfach ethylenisch ungesättigt sind. Bevorzugt verwendete ethylenisch ungesättigte Co-Monomere sind Acrylate oder Methacrylate. Besonders bevorzugte ethylenisch ungesättigte Co-Monomere sind Bis-GMA, TEGDMA, Bis-hydroxymethyl)tricyclo[5.2.1.$0^{2,6}$]-decandiacrylsäureester sowie 2,2-Bis[4,1-phenylenoxy(3,1-propandiyl)-methacrylsäureester]-propyliden.

Des weiteren können mindestens einfach epoxyfunktionalisierte Co-Monomere verwendet werden. Besonders bevorzugte epoxyfunktionalisierte Co-Monomere sind das in der DE-A-196 48 283 offenbarte 3,4-Epoxycyclohexylmethyl-3',4'-epoxycyclohexylcarboxylat und Tetrakis-[3,4-epoxycyclohexylethyl]-tetramethyltetracyclosiloxan.

Geeignete Füllstoffe als Bestandteil (b) sind in der Regel anorganische Füllstoffe. Beispielhaft genannt seien Quarz, gemahlene Gläser, Kieselgele sowie pyrogene Kieselsäuren oder deren Granulate. Bevorzugt werden röntgenopake Füllstoffe, zumindest teilweise, mit eingesetzt. Diese können zum einen röntgenopake Gläser sein, also Gläser, welche z.B. Strontium, Barium oder Lanthan enthalten, oder ein Teil der Füllkörper besteht aus einem röntgenopaken Zusatz, wie beispielsweise Yttriumtrifluorid, Strontiumhexafluorozirkonat oder Fluoriden der Selten-Erdmetalle.

Zur Verwendung in Pulver-Flüssigkeitssystemen eignen sich als Füllstoff insbesondere Gläser wie sie z.B in der EP-A-0 023 013 beschrieben sind. Zum besseren Einbau in die Polymermatrix ist es von Vorteil, die anorganischen Füllstoffe zu hydrophobieren. Übliche Hydrophobierungsmittel sind Silane, beispielsweise Trimethoxymethacryloyloxypropylsilan oder Trimethoxyglycidylsilan. Die Füllkörper haben vorzugsweise eine mittlere Kornverteilung <20 µm und insbesondere <5 µm, sowie eine obere Korngrenze von 150, vorzugsweise 70 µm und insbesondere 25 µm. Besonders bevorzugt werden Gemische von 5-25 Gew.-% Füllstoffe mit einer mittleren Korngröße von 0,02 - 0,06 µm und 65-85 Gew.-% Füllkörper mit einer mittleren Korngröße von 1-5 µm verwendet.

Als Katalysatoren der ROMP sind eine ganze Reihe von Übergangsmetallverbindungen bekannt, die je nach Anwendungszweck der ROMP verwendet werden. Für die erfindungsgemäßen Dentalmassen sind als Bestandteil (c) Verbindungen der folgenden allgemeinen Formel

$$WX_nL_{6-n}$$

in Kombination mit

$$R_mAlX_{3-m}$$

geeignet, wobei bedeuten:

X gleich F, Cl, Br, L ein $C_1$-$C_{20}$-Alkoholat oder $C_6$-$C_{20}$-Phenolat mit bis zu 5 Heteroatomen aus der Gruppe N, O, Si, P, S und bis zu fünffach mit Cl oder Br substituiert, R einen linearen oder verzweigten $C_1$-$C_{10}$-Alkylrest, n gleich 0-6 und m gleich 0-3, und eine Aktivierung gegebenenfalls durch Zinnalkyle mit $C_1$-$C_5$-Alkylresten erfolgen kann.

Ebenfalls verwendet werden können Verbindungen der allgemeinen Formel

$$L^1{}_aL^2{}_bM{=}CR^1R^2$$

wobei bedeuten:

M Mo, W, Ta oder Nb,
$L^1$ $OR^3$ oder Cl oder Br, wobei $R^3$ ein linearer oder verzweigter oder cyclischer $C_1$-$C_{10}$-Alkylrest ist, der teilweise oder vollständig fluoriert sein kann,
$L^2$ $PR^4{}_3$ oder N=Ar, wobei $R^4$ ein linearer oder verzweigter $C_1$-$C_{20}$-Alkylrest oder ein aromatischer $C_7$-$C_{20}$-Rest, und Ar ein aromatischer $C_7$-$C_{20}$-Rest ist, der durch lineares oder verzweigtes $C_1$-$C_{10}$-Alkyl, eine $NH_2$-Gruppe oder eine OH-Gruppe substituiert sein kann,
$R^1$ und $R^2$ H oder lineares oder verzweigtes oder cyclisches $C_1$-$C_{15}$-Alkyl oder $C_6$-$C_{15}$-Aryl oder $C_7$-$C_{15}$-Alkaryl sowie zusammen $C_1$-$C_{15}$-Alkenyl,
und a und b jeweils einen Wert von 0 bis 6, mit der Maßgabe $a + b \neq 0$.

Besonders bevorzugt sind Verbindungen in denen $R^3$ tertiäre Alkylreste bzw. in denen Ar aromatische Reste mit Alkylsubstituenten in 2,6-Stellung bedeuten.
Des weiteren sind als Bestandteil (b) Verbindungen der allgemeinen Formel

$$L^3{}_cL^4{}_dL^5{}_eRu({=}CR^5R^6)_f$$

geeignet, wobei bedeuten:

$L^3$ Cl, Br, Tosylat oder $R^7$, wobei $R^7$ ein linearer oder verzweigter oder cyclischer $C_1$-$C_{10}$-Allrylrest ist, der teilweise oder vollständig fluoriert sein kann,
$L^4$ $PR^8{}_3$ oder N=Ar, wobei $R^8$ ein linearer oder verzweigter $C_1$-$C_{20}$-Alkylrest oder ein aromatischer $C_7$-$C_{20}$-Rest und Ar ein aromatischer $C_7$-$C_{20}$-Rest ist, der durch lineares oder verzweigtes $C_1$-$C_{10}$-Alkyl, eine $NH_2$-Gruppe oder eine OH-Gruppe substituiert sein kann,
$L^5$ Benzol oder alkyl-substituierte $C_7$-$C_{20}$-Aromaten,
$R^5$ und $R^6$ H oder lineares oder verzweigtes oder cyclisches $C_1$-$C_{15}$-Alkyl oder $C_6$-$C_{15}$-Aryl oder $C_7$-$C_{15}$-Alkaryl sowie zusammen $C_1$-$C_{15}$-Alkylen,
und c, d, e und f jeweils einen Wert von 0 bis 4, mit der Maßgabe: $c + d + e + f \neq 0$.

Besonders bevorzugt sind Verbindungen, in denen $R^3$ tertiäre Alkylreste bzw. in denen Ar aromatische Reste mit Alkylsubstituenten in 2,6-Stellung bedeuten.
Die zuletzt genannten Katalysatoren eignenen sich auch für den Einsatz in wasserhaltigen Dentalmassen, sofern in der Dentalmasse mindestens 5 Gew.-% Wasser enthalten sind.
Für Dentalmassen, die einen solchen Wassergehalt aufweisen, eignen sich als Katalysatoren für die ROMP darüber hinaus auch Verbindungen der allgemeinen Formel

$$[L^3{}_cL^4{}_dL^5{}_eRu({=}CR^5R^6)_fX^1{}_g]^{h-}$$

wobei bedeuten:

$L^3$ Cl, Br, Tosylat oder $R^7$, wobei $R^7$ ein linearer oder verzweigter oder cyclischer $C_1$-$C_{10}$-Alkylrest ist, der teilweise oder vollständig fluoriert sein kann,
$L^4$ $PR^8{}_3$ oder N=Ar, wobei $R^8$ ein linearer oder verzweigter $C_1$-$C_{20}$-Alkylrest oder ein aromatischer $C_7$-$C_{20}$-Rest und Ar ein aromatischer $C_7$-$C_{20}$-Rest ist, der durch lineares oder verzweigtes $C_1$-$C_{10}$-Alkyl, eine $NH_2$-Gruppe oder eine OH-Gruppe substituiert sein kann,
$L^5$ Benzol oder alkyl-substituierte $C_7$-$C_{20}$-Aromaten,
$R^5$ und $R^6$ H oder lineares oder verzweigtes oder cyclisches $C_1$-$C_{15}$-Alkyl oder $C_6$-$C_{15}$-Aryl oder $C_7$-$C_{15}$-Alkaryl sowie zusammen $C_1$-$C_{15}$-Alkylen,
c, d, e und feinen Wert von 0 bis 4,

$X^1$ $L^3$ und

g und h jeweils einen Wert von 0 bis 3,

mit der Maßgabe: $c + d + e + f + g + h \neq 0$ .

Zusätzlich zu den genannten Katalysatoren, welche die ROMP auslösen, können als Bestandteil (c) noch weitere Katalysatoren enthalten sein, wie z.B. Radikalbildner oder Kationenbildner. Dies ist insbesondere dann der Fall, wenn die erfindungsgemäßen Dentalmassen durch die ROMP nur teilgehärtet und über einen zweiten Reaktionsmechanismus endgehärtet werden. Als radiakalbildende Katalysatoren können durch UV- oder sichtbares Licht aktivierbare Substanzen verwendet werden, wie z.B. Benzoinalkylether, Benzilketale, Acylphosphinoxide oder aliphatische und aromatische 1,2-Diketonverbindungen, z.B. Campherchinon, wobei die Lichtpolymerisation durch Zusatz von Aktivatoren, wie tertiären Aminen oder organischen Phosphiten, in an sich bekannter Weise beschleunigt werden kann.

Geeignete Initiatorsysteme zur Auslösung der radikalischen Polymerisation über einen Redox-Mechanismus sind beispielsweise die Systeme Peroxid/Amin oder Peroxid/Barbitursäurederivate u. dergl. Bei Verwendung solcher Initiatorsysteme ist es zweckmäßig, einen Initiator (z.B. Peroxid) und eine Katalysatorkomponente (z.B. Amin) getrennt bereitzuhalten. Die beiden Komponenten werden dann kurz vor ihrer Anwendung miteinander homogen vermischt.

Als Kationenbildner verwendet werden können Säurebildner wie z.B. Lewis oder Broensted-Säuren bzw. Verbindungen, die solche Säuren freisetzen, welche die kationische Polymerisation initiieren, beispielsweise $BF_3$ oder dessen etherische Addukte ($BF_3$*THF, $BF_3$*$Et_2O$, etc.), $AlCl_3$, $FeCl_3$, $HPF_6$, $HAsF_6$, $HSbF_6$, $HBF_4$ oder Substanzen, die nach Bestrahlen durch UV oder sichtbares Licht oder durch Wärme und/oder Druck die Polymerisation auslösen, wie z.B. (eta-6-Cumol)(eta-5-cyclopentadienyl)eisen-hexafluorophosphat, (eta-6-Cumol)(eta-5-cyclopentadienyl)eisen-tetrafluoroborat, (eta-6-Cumol)(eta-5-cyclopentadienyl)eisen-hexafluoroantimonat, substituierte Diaryliodoniumsalze und Triarylsulfonium-Salze, verwendet. Als Beschleuniger können Peroxyverbindungen vom Typ der Perester, der Diacylperoxide, der Peroxydicarbonate und der Hydroperoxide eingesetzt werden. Bevorzugt werden Hydroperoxide verwendet. Als besonders bevorzugter Beschleuniger kommt Cumolhydroperoxid in etwa 70-90 %iger Lösung in Cumol zum Einsatz. Das Verhältnis vom Photoinitiator zum Cumolhydroperoxid kann in weiten Grenzen von 1 : 0,001 bis 1 : 10 variiert werden, vorzugsweise wird jedoch ein Verhältnis von 1 : 0,1 bis 1 : 6 und besonders bevorzugt von 1 : 0,5 bis 1 : 4 verwendet. Die Verwendung von Komplexbildnern, wie beispielsweise Oxalsäure, 8-Hydroxychinolin, Ethylendiamintetraessigsäure und aromatischen Polyhydroxyverbindungen ist ebenfalls möglich. Als Verzögerer können Basen, typischerweise tertiäre Amine, zugesetzt werden.

Geeignete Hilfsstoffe gemäß Komponente (d) können beispielsweise üblicherweise auf dem Dentalgebiet eingesetzte Stabilisatoren, Pigmente oder Verdünnungsmittel sein.

Die erfindungsgemäßen Dentalmassen können sowohl als einkomponentige als auch als mehrkomponentige Systeme für den Anwender vorliegen. Im Falle von einkomponentigen Dentalmassen erfolgt die Aktivierung der darin enthaltenen Katalysatoren vorzugsweise mit Licht was in der Regel zur raschen Endhärtung der Dentalmassen führt. Hierzu liegen die Dentalmassen vorzugsweise pastenförmig vor. Bei mehrkomponentigen Systemen liegen die Massen in räumlich voneinander getrennten Behältnissen vor und werden erst unmittelbar vor ihrer Anwendung von Hand oder durch geeignete Mischhilfen miteinander vermischt. Durch die Vermischung wird der Aushärteprozess gestartet und führt zu einer Teil- oder Endhärtung der gesamten Masse. Hierzu können entweder Paste-Paste-Systeme oder Pulver-Flüssigkeits-Systeme verwendet werden. Wenn die Katalysatoren so gewählt werden, daß es nur zu einer Teilhärtung durch den Anmischvorgang kommt, so enthalten die Massen vorzugsweise noch weitere photochemisch aktivierbare Initiatoren, welche durch eine Belichtung der Massen in einem zweiten Schritt zur Endhärtung der Dentalmasse führen. Dadurch wird es für den Anwender möglich, die Dentalmasse im teilgehärteten Zustand zu bearbeiten und anschließend zur Anwendung z. B. im Mund des Patienten in die vollständig ausgehärtete Masse zu überführen.

Die erfindungsgemäßen Dentalmassen zeichnen sich durch einen raschen Polymerisationsvorgang aus, der zu teil- oder endgehärteten Materialien führt, welche einen geringen Volumenschrumpf, eine geringe Abrasionsneigung und gute mechanische Werte aufweisen.

Im Folgenden wird die Erfindung durch Beispiele genauer erläutert.

**Beispiele**

**Beispiel 1:** Einkomponentiges Füllungsmaterial, welches durch lichtinduzierte ROMP aushärtet

20 g Verbindung II werden mit 2,0 g [W(=NPh))CH2SiMe3)2{OCMe(CF3)2}2] versetzt und solange gerührt bis eine klare Lösung entsteht. Diese Lösung wird mit 0,5 g hochdispersem Siliciumdioxid (Aerosil OX50, Fa. Degussa) und 77,5 g feingemahlenem Quarzpulver verknetet, so daß eine homogene Paste entsteht. Zur Herstellung von Prüfkörpern zur Bestimmung der mechanischen Eigenschaften wird die Paste in entsprechende Prüfkörperformen eingebracht und gemäß den Angaben der ISO-Norm 4049 durch Licht ausgehärtet.

**Beispiel 2:** Zweikomponentiges Füllungsmaterial, welches durch ROMP aushärtet

4,0 g Verbindung VI werden mit 0,05 g hochdispersem Siliciumdioxid (Aerosil OX50, Fa. Degussa) und 5,95 g feingemahlenem Quarzpulver zu einer homogenen Paste A2 verknetet.

0,4 g ROMP-Katalysator auf Basis von Aryl-Ruthenat (CGI 452, Fa. Ciba Specialty Chemicals) werden mit 0,05 g hochdispersem Siliciumdioxid (Aerosil OX50, Fa. Degussa) und 3,0 g Dioctylphthalat und 6,55 g feingemahlenem Quarzpulver zu einer homogenen Paste B2 verknetet. Zur Herstellung von Prüfkörpern zur Bestimmung der mechanischen Eigenschaften werden je 1,0 g der Pasten A2 und B2 homogen miteinander vermengt, in entsprechende Prüfkörperformen eingebracht (gemäß ISO-Norm 4049) und nach dem Aushärten (ca. 5 Minuten) entformt.

**Beispiel 3:** Zweikomponentiges provisorisches Kronen- und Brückenmaterial, welches in einer zweiten Härtungsstufe radikalisch polymerisiert wird

4,6 g Bis-(hydroxymethyl)tricyclo[5.2.1.$0^{2,6}$]-decandiacrylsäureester werden mit 0,4 g ROMP-Katalysator auf Basis von Aryl-Ruthenat (CGI 452, Fa. Ciba Specialty Chemicals), 0,05 g hochdispersem Siliciumdioxid (Aerosil OX50, Fa. Degussa) und 4,95 g feingemahlenem Quarzpulver zu einer homogenen Paste A3 verknetet.

5,0 g Verbindung III werden mit 0,02 g Campherchinon, 0,05 g hochdispersem Siliciumdioxid (Aerosil OX50, Fa. Degussa) und 4,93 g feingemahlenem Quarzpulver zu einer homogenen Paste B3 verknetet.

Zur Anfertigung eines Provisoriums werden jeweils gleiche Mengen der Pasten A3 und B3 homogen miteinander gemischt und in die entsprechende Vorlage gegeben. Nach etwa 2-3 Minuten erreicht das Material eine Zwischenhärtung, die sich auch durch längeres Zuwarten nicht mehr verändert. Man belichtet das vorgehärtete Material 40 Sekunden lang mit sichtbarem Licht, um zur endgültigen Aushärtung zu gelangen.

**Beispiel 4:** Zweikomponentiges provisorisches Kronen- und Brückenmaterial, welches in einer zweiten Härtungsstufe kationisch polymerisiert wird

4,8 g Tetrakis-[3,4-Epoxycyclohexylethyl]-tetramethyltetracyclosiloxan, 0,4 g ROMP-Katalysator auf Basis von Aryl-Ruthenat (CGI 452, Fa. Ciba Specialty Chemicals), 0,05 g hochdispersem Siliciumdioxid (Aerosil OX50, Fa. Degussa) und 4,75 g feingemahlenem Quarzpulver zu einer homogenen Paste A4 verknetet.

5,0 g Verbindung III werden mit 0,02 g Ferroceniumhexafluoroantimonat, 0,02g Cumolhydroperoxid, 0,05 g hochdispersem Siliciumdioxid (Aerosil OX50, Fa. Degussa) und 4,91 g feingemahlenem Quarzpulver zu einer homogenen Paste B4 verknetet.

Zur Anfertigung eines Provisoriums werden jeweils gleiche Mengen der Pasten A4 und B4 homogen miteinander gemischt und in die entsprechende Vorlage gegeben. Nach etwa 2-3 Minuten erreicht das Material eine Zwischenhärtung, die sich auch durch längeres Zuwarten nicht mehr verändert. Man belichtet das vorgehärtete Material 40 Sekunden lang mit sichtbarem Licht, um zur endgültigen Aushärtung zu gelangen.

**Beispiel 5:** Pulver-Flüssigkeitssystem als dentales Füllmaterial

1,0 g Verbindung XI werden mit 1,0 g eines Glaspulvers (CHELON-FIL-Pulver, Fa. ESPE, Seefeld) gemischt. Dies Pulvermischung wird mit 0,6 g einer wässrigen Lösung $K_2RuCl_5*xH_2O$ (c= 140mg/ml) mit Hilfe eines Spatels angerührt. Zur Herstellung von Prüfkörpern zur Bestimmung der mechanischen Eigenschaften wird die Füllmasse unmittelbar nach dem Anmischen in entsprechende Prüfkörperformen gemäß der ISO-Norm 4049 bzw. ISO-Norm 9917 eingefüllt und abgewartet, bis die Massen abgebunden haben.

Tabelle 1

| Übersicht über die mechanischen Daten der erfindungsgemäßen Beispiele | | | | | |
|---|---|---|---|---|---|
| | Beispiel Nr.: | | | | |
| | 1 | 2 | 5 | Pertac II (Fa. ESPE, Seefeld) | Ketac-Fil (Fa. ESPE, Seefeld) |
| Druckfestigkeit [MPa] | 392[a)] | 405[a)] | 155[b)] | 420[a)] | 165[b)] |
| Biegefestigkeit [MPa] | 94[a)] | 92[a)] | 48[b)] | 100[a)] | 35[b)] |
| Volumenschrumpf [%][c)] | 1,4 | 1,1 | --- | 2,3 | ---- |
| Abrasion [µm][d)] | 30 | 35 | 87 | 34 | 94 |

a) Messung gemäß ISO-Norm 4049
b) Messung gemäß ISO-Norm 9917
c) Gemessen an ACTA-Linometer (A. J. deGee, A. J. Feilzer, C. L. Davidson; Dent. Mat. (1993), 9, 11-14)
d) Gemessen an ACTA-Abrasionsmaschine (A.J. de Gee, P. Pallav; J Dent 1994, 22 (1), 21-27)

## Patentansprüche

**1.** Dentalmasse enthaltend

(a) 5 bis 70 Gew.-%, bezogen auf (a) + (b) + (d) , polymerisierbare Monomere und/oder Polymere,
(b) 0 bis 95 Gew.-%, bezogen auf (a) + (b) + (d) , Füllstoffe,
(c) 0,01 bis 15 Gew.-%, bezogen auf (a), mindestens eines Initiators oder eines Initiatorsystems,
(d) 0 bis 95 Gew.-%, bezogen auf (a) + (b) + (d) , übliche Hilfsstoffe, einschließlich Pigmente, röntgenopake Zusatzstoffe und/oder Thixotropie-Hilfsmittel,

dadurch gekennzeichnet, daß der Bestandteil (a) eine solche chemische Struktur aufweist, daß die Teil- oder Endhärtung der Dentalmasse durch Ring-Öffnungs-Metathese-Polymerisation erfolgen kann.

**2.** Dentalmasse nach Anspruch 1, dadurch gekennzeichnet, daß sie die Bestandteile (a) bis (d) in den folgenden Mengenanteilen enthält:

(a) 15 bis 60 Gew.-%, bezogen auf (a) + (b) + (d)
(b) 40 bis 80 Gew.-%, bezogen auf (a) + (b) + (d)
(c) 2,0 bis 10 Gew.-%, bezogen auf (a), und
(d) 0 bis 30 Gew.-%, bezogen auf (a) + (b) + (d) .

**3.** Dentalmasse nach den Ansprüchen 1 und 2, dadurch gekennzeichnet, daß Bestandteil (a) Monomere der folgenden allgemeinen Formel

$$M\text{-}A_n$$

sind, wobei bedeuten:

M gleich H oder einen linearen, verzweigten, cyclischen oder polycyclischen organischen oder metallorganischen Rest, wobei die organischen Reste ausgewählt sein können aus $C_1$-$C_{30}$-Alkyl, $C_6$-$C_{20}$-Aryl, $C_7$-$C_{30}$-Alkaryl und $C_3$-$C_{30}$-Cycloalkyl mit 0-10 Heteroatomen aus der Gruppe N, O, Si, P, S und einer Anzahl von n Anknüpfungspunkten für A, und die metallorganischen Reste neben den oben genannten organischen Resten zusätzlich lineare, verzweigte, cyclische oder polycyclische Gerüste anorganischer Natur enthalten,

A einen ungesättigten cyclischen oder polycyclischen organischen Rest der allgemeinen Formel

$$C\text{-}D$$

wobei C ein linearer, verzweigter oder cyclischer gesättigter oder ungesättigter organischer $C_1$-$C_{20}$-Rest mit 0-10 Heteroatomen aus der Gruppe N, O, Si, P, S und 0-10 Carbonylgruppen ist und

D ein Cyclobutenyl-, Cyclopentenyl- oder ein an bezeichneter und wahlweise zusätzlich an einer weiteren Stelle im Ringsystem ein ungesättigter Rest der allgemeinen Formel

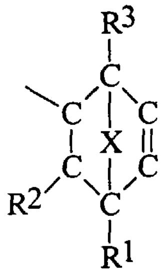

ist, in welcher bedeuten:

$R^1$, $R^2$, $R^3$ H oder einen linearen, verzweigten oder cyclischen gesättigten oder ungesättigten organischen $C_1$-$C_{20}$-Rest mit 0-10 Heteroatomen aus der Gruppe N, O, Si, P, S und 0-10 Carbonylgruppen, und

X O, NH, S bzw. einen gesättigten oder ungesättigten $C_1$-$C_{30}$-Kohlenwasserstoffrest.

**4.** Dentalmasse nach den Ansprüchen 1 bis 3, dadurch gekennzeichnet, daß Bestandteil (c) Verbindungen der allgemeinen Formel

$$WX_nL_{6-n}$$

in Kombination mit

$$R_mAlX_{3-m}$$

sind, wobei bedeuten:

X gleich F, Cl, Br,
L ein $C_1$-$C_{20}$-Alkoholat oder $C_6$-$C_{20}$-Phenolat mit bis zu 5 Heteroatomen aus der Gruppe N, O, Si, P, S und bis zu fünffach mit Cl oder Br substituiert,
R einen linearen oder verzweigten $C_1$-$C_{10}$-Alkylrest,
n gleich 0-6 und m gleich 0-3,
wobei die Dentalmasse zusätzlich als Aktivator ein Zinnalkyl mit $C_1$-$C_5$-Alkylresten enthalten kann.

**5.** Dentalmasse nach den Ansprüchen 1 bis 3, dadurch gekennzeichnet, daß Bestandteil (c) Verbindungen der allgemeinen Formel

$$L^1_aL^2_bM=CR^1R^2$$

sind, wobei bedeuten:

M Mo, W, Ta oder Nb,
$L^1$ $OR^3$ oder Cl oder Br, wobei $R^3$ ein linearer oder verzweigter oder cyclischer $C_1$-$C_{10}$-Alkylrest ist, der teilweise oder vollständig fluoriert sein kann,
$L^2$ $PR^4_3$ oder N=Ar, wobei $R^4$ ein linearer oder verzweigter $C_1$-$C_{20}$-Alkylrest oder ein aromatischer $C_7$-$C_{20}$-Rest und Ar ein aromatischer $C_7$-$C_{20}$-Rest ist, der durch lineares oder verzweigtes $C_1$-$C_{10}$-Alkyl, eine $NH_2$-Gruppe oder eine OH-Gruppe substituiert sein kann,
$R^1$ und $R^2$ H oder lineares oder verzweigtes oder cyclisches $C_1$-$C_{15}$-Alkyl oder $C_6$-$C_{15}$-Aryl oder $C_7$-$C_{15}$-Alkaryl sowie zusammen $C_1$-$C_{15}$-Alkylen,
und a und b jeweils einen Wert von 0-6, mit der Maßgabe: $a + b \neq 0$ .

6. Dentalmasse nach den Ansprüchen 1 bis 3, dadurch gekennzeichnet, daß Bestandteil (c) Verbindungen der allgemeinen Formel

$$L^3_cL^4_dL^5_eRu(=CR^5R^6)_f$$

sind, wobei bedeuten:

$L^3$ Cl, Br, Tosylat oder $R^7$, wobei $R^7$ ein linearer oder verzweigter oder cyclischer $C_1$-$C_{10}$-Alkylrest ist, der teilweise oder vollständig fluoriert sein kann,
$L^4$ $PR^8_3$ oder N=Ar, wobei $R^8$ ein linearer oder verzweigter $C_1$-$C_{20}$-Alkylrest oder ein aromatischer $C_7$-$C_{20}$-Rest und Ar ein aromatischer $C_7$-$C_{20}$-Rest ist, der durch lineares oder verzweigtes $C_1$-$C_{10}$-Alkyl, eine $NH_2$-Gruppe oder eine OH-Gruppe substituiert sein kann,
$L^5$ Benzol oder alkyl-substituierte $C_7$-$C_{20}$-Aromaten,
$R^5$ und $R^6$ H oder lineares oder verzweigtes oder cyclisches $C_1$-$C_{15}$-Alkyl oder $C_6$-$C_{15}$-Aryl oder $C_7$-$C_{15}$-Alkaryl sowie zusammen $C_1$-$C_{15}$-Alkylen, und
c, d, e und f jeweils einen Wert von 0 bis 4, mit der Maßgabe: $c + d + e + f \neq 0$ .

7. Dentalmasse nach Anspruch 6, dadurch gekennzeichnet, daß zusätzlich mindestens 5 Gew.-% Wasser in der Dentalmasse vorhanden sind.

8. Dentalmasse nach den Ansprüchen 1 bis 3, dadurch gekennzeichnet, daß Bestandteil (c) Verbindungen der allgemeinen Formel

$$[L^3_cL^4_dL^5_eRu(=CR^5R^6)_fX^1_g]^{h-}$$

sind, wobei bedeuten:

$L^3$ Cl, Br, Tosylat oder $R^7$, wobei $R^7$ ein linearer oder verzweigter oder cyclischer $C_1$-$C_{10}$-Alkylrest ist, der teilweise oder vollständig fluoriert sein kann,
$L^4$ $PR^8_3$ oder N=Ar, wobei $R^8$ ein linearer oder verzweigter $C_1$-$C_{20}$-Alkylrest oder ein aromatischer $C_7$-$C_{20}$-Rest und Ar ein aromatischer $C_7$-$C_{20}$-Rest ist, der durch lineares oder verzweigtes $C_1$-$C_{10}$-Alkyl, eine $NH_2$-Gruppe oder eine OH-Gruppe substituiert sein kann,
$L^5$ Benzol oder alkyl-substituierte $C_1$-$C_{20}$-Aromaten,
$R^5$ und $R^6$ H oder lineares oder verzweigtes oder cyclisches $C_1$-$C_{15}$-Alkyl oder $C_6$-$C_{15}$-Aryl oder $C_7$-$C_{15}$-Alkaryl sowie zusammen $C_1$-$C_{15}$-Alkylen,
c, d, e und f jeweils einen Wert von 0 bis 4,
$X^1$ $L^3$, und
g und h jeweils einen Wert von 0 bis 3,
mit der Maßgabe: daß $c + d + e + f + g + h \neq 0$ und mit der Maßgabe, daß 5-Gew.-% Wasser, bezogen auf Bestandteil (c), in der Dentalmasse vorhanden sind.

9. Dentalmasse nach den Ansprüchen 1 bis 8, dadurch gekennzeichnet, daß sie zusätzlich einen Initiator für die Endhärtung in einer zweiten Stufe durch radikalische Polymerisation enthält.

10. Dentalmasse nach den Ansprüchen 1 bis 8, dadurch gekennzeichnet, daß sie zusätzlich einen Initiator für die Endhärtung in einer zweiten Stufe durch kationische Polymerisation enthält.

11. Verwendung der Dentalmasse nach den Ansprüchen 1 bis 10 zur Herstellung von polymerisierbaren Füllungsmaterialien, Befestigungszementen, Bondingmischungen, Inlays, Onlays, Verblendschalen, provisorischen Kronen- und Brückenmaterialien, zahntechnischen Werkstoffen, Modellmaterialien sowie Abformmaterialien.